# EUROPEAN PATENT APPLICATION

(11) **EP 3 447 515 A1**
(43) Date of publication of application: **27.02.2019**
(21) Application number: 17187453.0
(22) Date of filing: 23.08.2017
(51) Int. Cl.: G01R 33/24, G01R 33/44, A61B 5/053, G01R 33/567, G01R 33/48

(54) **MAGNETIC RESONANCE IMAGING WITH A VARIABLE FIELD MAGNET**

(71) Applicant: Koninklijke Philips N.V., 5656 AE Eindhoven (NL)
(72) Inventor: GLEICH, Bernhard, 5656 AE Eindhoven (NL)
(74) Representative: de Haan, Poul Erik

(57) **Abstract**

The invention provides for a magnetic resonance imaging (MRI) (100) system comprising a main magnet (102) with an with an adjustable main magnetic field. The MRI system further comprises a current source (124) for supplying RF current between multiple electrodes (122, 122') divided between a first portion (122) and a second portion (122'). The current source is configured for supplying the RF current between the first portion and the second portion. Execution of the machine executable instructions cause a processor controlling the MRI system to: set (200) the average magnetic field strength within the imaging zone to a first value; set (202) the average magnetic field strength within the imaging zone to a second value, the second value is lower than the first value; control (204) the current source to have a known RF current (144) travel between the first portion of the electrodes and the second portion of the electrodes; acquire (206) the magnetic resonance data from the subject by controlling the magnetic resonance imaging system with readout gradient commands according to a three-dimensional imaging protocol; reconstruct (208) three-dimensional image data (148) from the magnetic resonance data; and calculate (210) a resistive model (150) of the subject using the three-dimensional image data and the known RF current through the electrodes.

## Description

### TECHNICAL FIELD OF THE INVENTION

The invention relates to magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI. For example various electrical properties of a subject can be investigated using MRI.

United States patent application US 2015/0153431 discloses systems and methods for determining electrical properties using Magnetic Resonance Imaging (MRI). One method includes applying an ultra-short echo time (TE) pulse sequence in a Magnetic Resonance Imaging (MRI) system and acquiring a complex B1⁺ B1⁻ quantity from an object following the application of the ultra-short TE pulse sequence, where B1⁺ is a complex amplitude of a transmit radio-frequency (RF) magnetic field and B1⁻ is a complex amplitude of a receive RF magnetic field. The method also includes estimating, with a processor, one or more electrical properties of the object using the complex amplitudes of the transmit RF magnetic field and the receive RF magnetic field.

### SUMMARY OF THE INVENTION

The invention provides for a magnetic resonance imaging system, a computer program product and a method in the independent claims. Embodiments are given in the dependent claims.

Embodiments may provide for an improved means of calculating a resistive model of a subject with magnetic resonance imaging. This resistive model may also be considered to be a spatially dependent mapping of the resistance within a subject. Such a resistive model could be used, for example, to study the electrophysiology of the heart. The resistive model is measured using a magnetic resonance imaging system which has a main magnet with an adjustable magnetic field in its imaging zone. The magnetic field in the imaging zone is first held at a first value to magnetize spins in the imaging zone. Next the magnetic field in the imaging zone is then lowered to a second value lower than the first value. Then surface electrodes are used to drive an RF current (at the current Larmor frequency), through the subject. This generates a flip angle in the spins being imaged. Then the magnetic field is optionally increased to a third value that is between the first value and the second value before readout. The changing of the magnetic field in this way may have the advantage that it enables measurement of the electrical impedance at a lower frequency which may facilitate the construction of resistive model.The electrical conductivity of tissue may vary considerably with the frequency. As the magnetic field, and the Larmor frequency, decreases the capacitive effect of the cell membrane decreases. The resistive model therefore may become more accurate. Lowering the frequency however, increases the chance for nerve stimulation, often times, the second value of the magnetic field strength is chosen such that the Larmor frequency is lowered to a compromise frequency that balances the accuracy of the resistive model against the stimulation of nerve tissue by the RF current.

The choice of the first value of the average magnetic field in the imaging zone is not critical. The first value is higher than the second value so that the spins within the imaging zone become polarized. As the average magnetic field lowers from the first value to the second value, some of the polarization is lost. One way to choose the value of the first value is to choose it so that it maximizes the polarization one the second value of the average magnetic field strength is reached. The choice of the first value is therefore dependent upon how quickly the magnetic field in the imaging zone can ramp between the first and second values.

In one aspect, the invention provides for a magnetic resonance imaging system which comprises a main magnet with an imaging zone. The main magnet is configured for generating a main magnetic field with an average magnetic field strength within the imaging zone. The magnetic resonance imaging system further comprises a gradient magnetic field system for generating a spatially dependent gradient magnetic field within the imaging zone. The gradient magnetic field system typically comprises a number of gradient magnetic field coils and a power supply for supplying current to those coils. The magnetic resonance imaging system further comprises a magnet power supply configured for adjusting the average magnetic field strength within the imaging zone. The inclusion of the magnet power supply effectively makes the magnetic field strength within the imaging zone adjustable.

The magnetic resonance imaging system further comprises a current source for supplying RF current between multiple electrodes. The multiple electrodes comprise a first portion and a second portion. The current source is configured for supplying the RF current between the first portion and the second portion. The multiple electrodes are configured for forming an electrical contact with an exterior surface of a subject. The magnetic resonance imaging system further comprises a memory containing machine-executable instructions and pulse sequence commands. The pulse sequence commands comprise instructions for controlling the magnetic resonance imaging system for acquiring magnetic resonance data from the imaging zone according to a three-dimensional imaging protocol. The pulse sequence commands comprise readout gradient commands for controlling the gradient magnetic field system.

The magnetic resonance imaging system further comprises a processor for controlling the magnetic resonance imaging system. The execution of the machine-executable instructions cause the processor to set the average magnetic field strength within the imaging zone to a first value by controlling the magnetic power supply with the pulse sequence commands. Execution of the machine-executable instructions then causes the processor to set the average magnetic field strength within the imaging zone to a second value by controlling the magnetic power supply with the pulse sequence commands. The second value is lower than the first value. Execution of the machine-executable instructions then causes the processor to control the current source to have a known RF current travel between the first portion of the electrodes and the second portion of the electrodes. This is done to tilt spins within the subject and within the imaging zone to an angle with respect to the main magnetic field. Execution of the machine-executable instructions further cause the processor to acquire the magnetic resonance data from the subject by controlling the magnetic resonance imaging system with the readout gradient commands.

The magnetic resonance imaging system may comprise an RF system which comprises a magnetic resonance imaging coil and a receiver for receiving the magnetic resonance data. Execution of the machine-executable instructions further cause the processor to reconstruct three-dimensional image data from the magnetic resonance data. Execution of the machine-executable instructions further cause the processor to calculate a resistive model of the subject using the three-dimensional image data and the known RF current through the electrodes. The known RF current can be directly related to the intensity or amplitude of the three-dimensional image date. This embodiment may be beneficial because it may provide for a means of making a non-invasive resistive model or measurements of a subject.

When the current source is controlled to have the known RF current travel between the first portion electrodes and the second portion electrodes the RF current may be applied at the Larmor frequency of some spins within the main magnetic field. Typically magnetic resonance imaging looks at the proton density so the Larmor frequency will be for hydrogen atoms.

It should be noted that the main magnet in combination with the magnet power supply effectively makes a main magnet with an adjustable magnetic field. The main magnet could for example be a superconducting magnet and the current source is simply used to adjust the magnetic field. In another example, the main magnet is a resistive type magnet and the current source continually supplies current to generate the average magnetic field within the imaging zone. In another example, the main magnet is a permanent magnet and the magnetic field strength is achieved by physically moving or rotating the magnet.

In another embodiment, the magnetic resonance imaging system is a Zero Echo Time (ZTE) magnetic resonance imaging system.

In another embodiment, the magnetic resonance imaging system is configured for receiving an ECG signal from the subject. Execution of the machine-executable instructions further comprises calculating a heart electrical potential of the subject using the resistive model, the ECG signal, and an electrical source model. The electrical source model could for example be a source of the electrical energy generated by the heart as it pumps. An electrical source model could for example be fit to the subject using the three-dimensional image date or other magnetic resonance image data that was acquired for the subject. This embodiment may be beneficial because it may provide for a non-invasive means of measuring potentials at the subject's heart. This may eliminate the need or benefit of inserting a catheter for making these measurements.

In some embodiments, the ECG electrodes are identical or partially identical with the multiple electrodes. In other examples the ECG electrodes may be separate or partially separate from the multiple electrodes.

In another embodiment, execution of the readout gradient commands is at least partially triggered by the ECG signal. This embodiment may be beneficial because it may provide for a means of acquiring the magnetic resonance data multiple times at the same phase of the heart. This could for example enable the calculation of a resistive model or a heart electrical potential that is resolved in terms of the phase of the heart. It may also enable very accurate measurements of the resistive model or the heart electrical potential for a particular phase of the heart motion.

In another embodiment, the current source comprises a current sensor to individually measure an RF electrode current for each of the multiple electrodes. The known RF current is determined using the RF electrode current for each of the multiple electrodes. This embodiment may be beneficial because then the current through each particular electrode may be mapped or determined very accurately. This may help in calculating a more accurate resistive model.

In another embodiment, the resistive model is calculated using a first finite difference model and a second finite difference model. The resistive model is effectively calculated in two stages. The first finite difference model is configured for solving for a current flow through the subject using the RF electrode current for each of the multiple electrodes and the amplitude and phase of the three-dimensional image data. The amplitude of the three-dimensional image data is effectively related to the current travelling locally through the subject. The first finite difference model is configured for calculating a current flow through the subject using a first optimization algorithm to optimize a first objective function. The first objective function fits the current flow to the intensity of the three-dimensional image using the Biot-Savart law. The Biot-Savart law relates a current flow to a generated magnetic field.

The magnetic field generated by the current flowing through the subject is directly responsible for tipping magnetic spins and is therefore directly related to the amplitude of the three-dimensional image data. Once the local current flow is calculated this is used as input to the second finite difference model. The second finite difference model fits the resistive model to the current flow using a second objective function. The second objective function fits the resistive model to the current flow using Ohm's law and the RF electrode current for each of the multiple electrodes. This embodiment may be beneficial because it provides for an effective means of calculating the resistive model of the subject. It should be noted that the first finite difference model and the second finite difference model are solved as optimization problems. The process can be repeated multiple times, that is to say different combinations of current flow through the multiple electrodes can be used and the process can be repeated. The data from these repeated experiments can all be combined into the first and second objective functions. Repeating the experiment using different current flows through different combinations of the electrodes may therefore lead to greatly increased knowledge of the resistive model of the subject.

In another embodiment, the current source is configured for switching the multiple electrodes between the first portion and the second portion. The machine-executable instructions further cause the processor to reconstruct the three-dimensional image data for the multiple permutations of the multiple electrodes distributed between the first portion and the second portion. The first objective function and the second objective function combine data from the multiple permutations of the multiple electrodes. In this embodiment the data from multiple experiments where different combinations of current flowing through the different electrodes is used. As mentioned above, this may be used to increase the accuracy and reduce the error in the resistive model.

In another embodiment, execution of the machine-executable instructions further cause the processor to control the current source to acquire electrical impedance tomography data using the multiple electrodes. The second objective function further fits the resistive model to the electrical impedance tomography data. This embodiment may be beneficial because it may further improve the quality of the resistive model.

The multiple electrodes may be used in an electrical impedance tomography mode. In electrical impedance tomography a DC or AC current is applied between at least two electrodes and the potential difference to all the other electrodes is measured. Then a different pair of electrodes serve as a current source and sink and the measurements at the rest of the electrodes is repeated. This may be done multiple times. The electrical impedance tomography is in principle known, but has a very low spatial resolution. The spatial resolution is the order of the distance to the surface of a given voxel. With a normal adult human this may result in a resolution of about 5 cm at the heart.

Nevertheless, the electrical impedance tomography data may be very useful for the magnetic resonance imaging reconstruction of the resistive model. This is because it may provide an average of absolute resistivity values and the errors that may possibly be made due to for example the relaxation of the spins which is different for different tissues could be at least partially compensated for. The electrical impedance tomography may also be helpful in extrapolating the results to even lower frequencies. The current supplied in electrical impedance tomography mode can be very small and does not hurt the patient even when using DC current. The resistive model may also be modified such that the data acquired in electrical impedance tomography is used to perform the optimization on the second finite difference model. Additional error terms can simply be added to the second objective function.

In another embodiment, the magnetic resonance imaging system further comprises a garment. The garment comprises the multiple electrodes. For example when imaging the chest the garment can simply be worn by a subject and all the multiple electrodes are effectively distributed across the surface of the subject in a useful fashion.

In some embodiments, the multiple electrodes are configured for contacting the exterior surface of the subject when the garment is worn.

In another embodiment, the pulse sequence commands are any one of the following: a spin echo pulse sequence commands, ZTE pulse sequence commands, an EPI pulse sequence commands, radially sampled pulse sequence commands and pulse sequence commands which comprise a spiral readout gradient sequence. This embodiment is beneficial because any one of these various types of pulse sequence commands may be used to acquire the magnetic resonance data. It should be noted that the pulse sequences above lack an RF pulse to initially tip over the spins. This is done by the current when the magnet is in the second state when the average magnetic field strength within the imaging zone is set to a second value.

In another embodiment, the second value of the average magnetic field strength is chosen such that the Larmor frequency is between 20 kHz and 200 kHz. This embodiment may be beneficial because the currents which are used will not result in a sensation of pain by the subject.

In another embodiment, the second value of the average magnetic field strength is between 5 mTesla and 0.2 mTesla. This embodiment may be beneficial because the currents which are passed through the subject through the multiple electrodes will not result in pain.

In another embodiment, execution of the machine executable instructions cause the processor to maintain the average magnetic field strength within the imaging zone at the first value for any one of the following: at least 10 ms, at least 20 ms, at least 100 ms, at least 300 ms, and at least 500 ms before setting the average magnetic field strength within the imaging zone to the second value. This embodiment may be beneficial the spins within the imaging zone may become magnetically polarized during this time. After the polarization has increased sufficiently, the magnetic field is lowered to the second value.

In another embodiment, execution of the machine executable instructions cause the processor set the average magnetic field strength within the imaging zone to a third value by controlling the magnet power supply with the pulse sequence commands before acquiring the magnetic resonance data from the subject. The third value is between the first value and the second value.

In another embodiment, the third value of the average magnetic field strength is between 25 mTesla and 150 mTesla.

In another embodiment execution of the machine executable instructions cause the processor to acquire the magnetic resonance data from the subject by controlling the magnetic resonance imaging system with the readout gradient commands while the average magnetic field strength within the imaging zone is set at the second value. This embodiment may be advantageous because it minimizes the delay before measurement. The degree of polarization may therefore be higher.

In another embodiment the known RF current has a current density on the electrodes less than any one of the following: 10 A/m², 5 A/m², 2 A/m², 1 A/m², and 0.5 A/m².

In another embodiment, the magnetic resonance imaging system is further configured for receiving a respiratory signal. Acquisition of the magnetic resonance data is at least partially triggered by the respiratory signal. This may be beneficial because it may enable the subject being in the same position when the magnetic resonance data is acquired multiple times.

In another embodiment, the multiple electrodes comprise magnetic resonance fiducial markers. Execution of the machine-executable instructions further causes the processor to register a location of each of the multiple electrodes to the three-dimensional image data by attaching a fiducial marker signal in the three-dimensional image data. The resistive model is further calculated using the location of each of the multiple electrodes. This embodiment may be beneficial because the current through each of the electrodes can be more accurately related to the three-dimensional imaging protocol. This may result in more accurate determination of the resistive model. It should be noted that the respiratory sensor could be part of an MRI system or an external system which just provides a signal such as a gate signal or breathing phase to the magnetic resonance imaging system. The respiratory sensor could for example be one of a sensor in a breathing tube or an external optical sensor such as a camera that looks at the chest of the subject and/or the motion of the fiducial markers. The respiratory sensor may also be a breathing belt that is used to measure the expansion and contraction of the thorax of the subject.

In another aspect, the invention provides for a computer program product comprising machine-executable instructions for execution by a processor controlling the magnetic resonance imaging system. The magnetic resonance imaging system comprises a main magnet with an imaging zone. The main magnet is configured for generating a main magnetic field with an average magnetic field strength within the imaging zone. The magnetic resonance imaging system further comprises a gradient magnetic field system for generating a spatially dependent gradient magnetic field within the imaging zone. The magnetic resonance imaging system further comprises a magnet power supply configured for adjusting the average magnetic field strength within the imaging zone. The magnetic resonance imaging system further comprises a current source for supplying RF current between multiple electrodes. The multiple electrodes comprise a first portion and a second portion. The current source is configured for supplying the RF current between the first portion and the second portion. The multiple electrodes are configured for forming an electrical contact with an exterior surface of a subject.

Execution of the machine-executable instructions cause the processor to set the average magnetic field strength within the imaging zone to a first value by controlling the magnetic power supply with pulse sequence commands. The pulse sequence commands comprise instructions for controlling the magnetic resonance imaging system for acquiring magnetic resonance data from the imaging zone according to a three-dimensional imaging protocol. The pulse sequence commands comprise readout gradient commands for controlling the gradient magnetic field system. Execution of the machine-executable instructions further cause the processor to set the average magnetic strength within the imaging zone to a second value by controlling the magnetic power supply with the pulse sequence commands. The second value is lower than the first value. Execution of the machine-executable instructions further cause the processor to control the current source to have a known RF current travel between the first portion of the electrodes and the second portion of the electrodes.

Execution of the machine-executable instructions further causes the processor to acquire the magnetic resonance data from the subject by controlling the magnetic resonance imaging system with the readout gradient commands. Execution of the machine-executable instructions further cause the processor to reconstruct three-dimensional image data from the magnetic resonance data. Execution of the machine-executable instructions further cause the processor to calculate a resistive model of the subject using the three-dimensional image data and the known RF current through the electrodes.

In another aspect, the invention provides for a method of operating the magnetic resonance imaging system. The magnetic resonance imaging system comprises a main magnet with an imaging zone. The main magnet is configured for generating a main magnetic field with an average magnetic field strength within the imaging zone. The magnetic resonance imaging system further comprises a gradient magnetic field system for generating a spatially dependent gradient magnetic field within the imaging zone. The magnetic resonance imaging system further comprises a magnet power supply configured for adjusting the average magnetic field strength within the imaging zone. The magnetic resonance imaging system further comprises a current source for supply RF current between multiple electrodes. The multiple electrodes comprise a first portion and a second portion. The current source is configured for supplying the RF current between the first portion and the second portion. The multiple electrodes are configured for performing an electrical contact with an exterior surface of a subject. The method comprises setting the average magnetic field strength within the imaging zone to a first value by controlling the magnet power supply with the pulse sequence commands. The pulse sequence commands comprise instructions for controlling the magnetic resonance imaging system for acquiring magnetic resonance data from the imaging zone according to a three-dimensional imaging protocol. The method further comprises setting the average magnetic field strength within the imaging zone to a second value by controlling the magnet power supply with the pulse sequence commands. The second value is lower than the first value. The pulse sequence commands comprise readout gradient commands for controlling the gradient magnetic field system. The method further comprises controlling the current source to have a known RF current travel between the first portion of the electrodes and the second portion of the electrodes. The method further comprises acquiring the magnetic resonance data from the subject by controlling the magnetic resonance imaging system with the readout gradient commands. The method further comprises reconstructing three-dimensional image data from the magnetic resonance data. The method further comprises calculating a resistive model of the subject using the three-dimensional image data and the known RF current through the electrodes.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. MRF magnetic resonance data is magnetic resonance data. Magnetic resonance data is an example of medical image data. A Magnetic Resonance Imaging (MRI) image or MR image is defined herein as being the reconstructed two or three dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system;
Fig. 2 shows a flow chart which illustrates a method of operating the magnetic resonance imaging system of Fig. 1;
Fig. 3 illustrates an example of a garment; and
Fig. 4 illustrates a further example of a garment.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

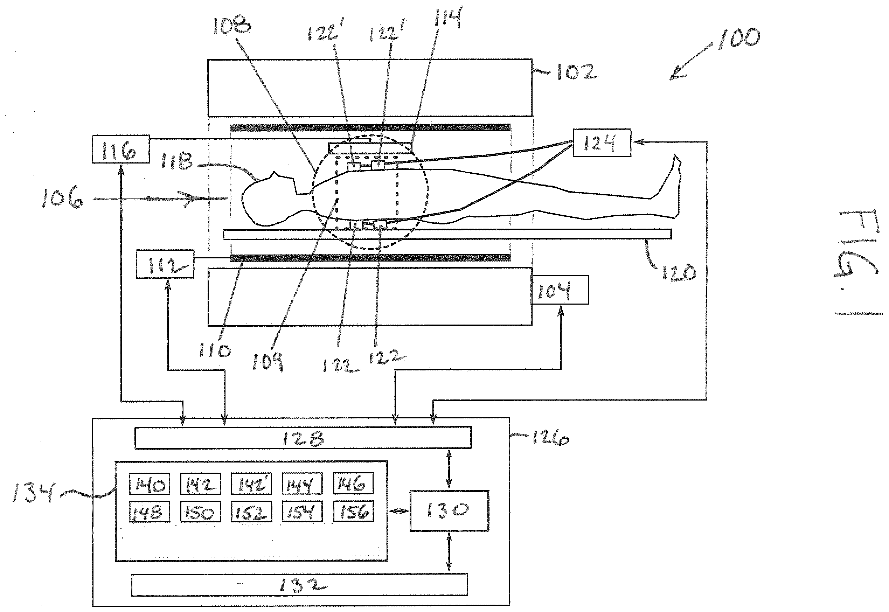

Fig. 1 illustrates an example of a magnetic resonance imaging system 100. The magnetic resonance imaging system comprises a magnet 102. A magnet power supply 104 is shown as being attached to the magnet 102. The magnet power supply 104 is able to change the main magnetic field average value within the imaging zone 108. The magnet 102 may for example be a superconducting magnet and the magnet power supply 104 is used to adjust the average value of the main magnetic field in the imaging zone 108. Alternatively the magnet 102 may be a resistive type magnet and the magnet power supply 104 supplies power continuously to generate the main magnetic field within imaging zone 108.

The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 102 there is an imaging zone 108 where the magnetic field is strong and uniform enough to perform magnetic resonance imaging. A region of interest 109 is shown within the imaging zone 108. A subject 118 is shown as being supported by a subject support 120 such that at least a portion of the subject 118 is within the imaging zone 108 and the region of interest 109.

Within the imaging zone 108 can be seen a number of electrodes 122, 122'. They are connected to a current source 124. In this example two of the electrodes are the first portion 122 and the other two electrodes are part of the second portion 122'. The current source 124 supplies current between the first portion 122 and the second portion 122'. The electrodes 122, 122' are connected to an external surface of the subject 118.

Within the bore 106 of the magnet there is also a set of magnetic field gradient coils 110 which is used for acquisition of preliminary magnetic resonance data to spatially encode magnetic spins within the imaging zone 108 of the magnet 102. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for receiving radio transmissions from spins also within the imaging zone 108. In some examples, the radio-frequency coil may also be configured for manipulating the orientations of magnetic spins within the imaging zone 108. The radio frequency antenna may contain multiple coil elements. The radio frequency antenna may also be referred to as a channel or antenna. The radio-frequency coil 114 is connected to a radio frequency reciever or transceiver 116. The radio-frequency coil 114 and radio frequency transceiver 116 may be optionally replaced by separate transmit and receive coils and a separate transmitter and receiver. It is understood that the radio-frequency coil 114 and the radio frequency transceiver 116 are representative. The radio-frequency coil 114 could also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

The transceiver 116, the gradient controller 112, current source 124, and magnet power supply 104 are shown as being connected to a hardware interface 128 of a computer system 126. The computer system further comprises a processor 130 that is in communication with the hardware system 128, a memory 134, and a user interface 132. The memory 134 may be any combination of memory which is accessible to the processor 130. This may include such things as main memory, cached memory, and also non-volatile memory such as flash RAM, hard drives, or other storage devices. In some examples, the memory 130 may be a non-transitory computer-readable medium.

The computer memory 134 is shown as containing machine-executable instructions 140 which may be used by the processor 130 to control the operation of the magnetic resonance imaging system 100 as various components. The computer memory 134 is further shown as containing pulse sequence commands 142. The pulse sequence commands 142 comprise readout gradient commands 142' for controlling the magnetic field gradient coil power supply 112. The computer memory 134 is further shown as containing a known or measured RF current 144 through the electrodes 122, 122'. The computer memory 134 is further shown as containing magnetic resonance data 146 that was acquired by controlling the magnetic resonance imaging system 100 with the pulse sequence commands 142. The computer memory 134 is further shown as containing three-dimensional image data 148 that was reconstructed from the magnetic resonance data 146. The computer memory further comprises a resistive model 150 that was reconstructed from the three-dimensional image data 148 and the known or measured RF current 144. The resistive model 150 is a spatially dependent model of the subject 118 which describes the local resistance. The computer memory 134 is further shown as optionally containing a first finite difference model 152, a current flow mapping 154, and a second finite difference model 156. The first finite difference model 152 uses the three-dimensional image data 148 and the known or measured RF current 144 to calculate the current flow mapping 154. The second finite difference model may use the current flow mapping 154 and the known or measured RF current 144 to calculate the resistive model 150.

In some examples all or some of the electrodes 122, 122' may also function as ECG electrodes. In some examples, the current source 124 may also incorporate an ECG system for obtaining an ECG signal from the electrodes 122, 122'.

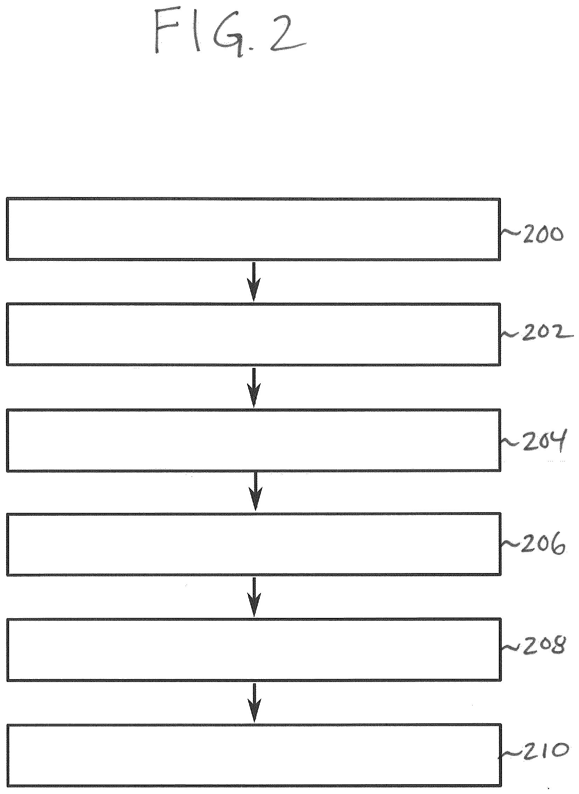

Fig. 2 shows a flowchart which illustrates a method of operating the magnetic resonance imaging system 100 depicted in Fig. 1. First in step 200 the average magnetic field strength is set within the imaging zone 108 to a first value by controlling the magnet power supply 104 with the pulse sequence commands 142. The pulse sequence commands 142 comprise instructions for controlling the magnetic resonance imaging system for acquiring the magnetic resonance image data from the imaging zone according to a three-dimensional imaging protocol. The pulse sequence commands comprise the readout gradient commands 142'. Then in step 202, the average magnetic field strength is set within the imaging zone 108 to a second value by controlling the magnetic power supply 104 with the pulse sequence commands. The second value is lower than the first value. Next in step 204 the current source 124 is controlled to have a known RF current 144 travel between the first portion of the electrodes 122 and the second portion of the electrodes 122'. Next in step 206 the magnetic resonance data 146 is acquired from the subject 118 by controlling the magnetic resonance imaging system 100 with the readout gradient commands 142'. Then in step 208 the three-dimensional image data 148 is reconstructed from the magnetic resonance data 146. Finally in step 210, the resistive model 150 is calculated using the three-dimensional image data 148 and the known RF current 144 through the electrodes 122, 122'.

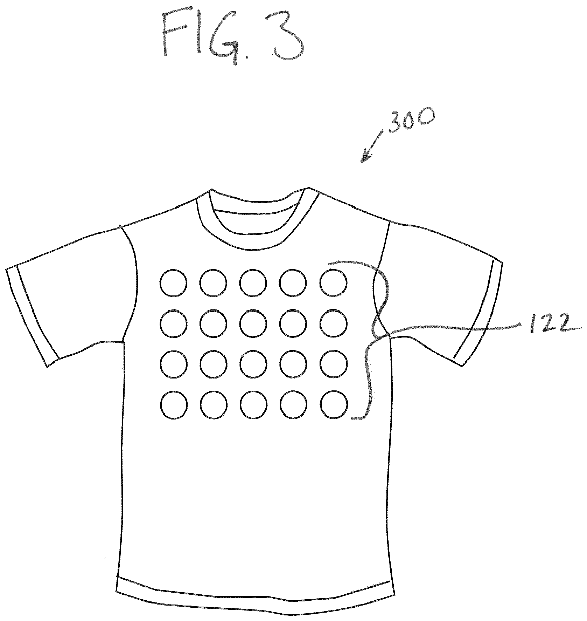

Fig. 3 shows an example of a garment 300 that the subject 118 could wear. There are a number of electrodes 122 which are mounted on the garment 300. When the subject 118 wears the garment 300 the electrodes 122 are automatically in contact with the subject's external surface 118. The view if Fig. 3 only shows a front portion of the garment. The back portion of the garment may also have electrodes.

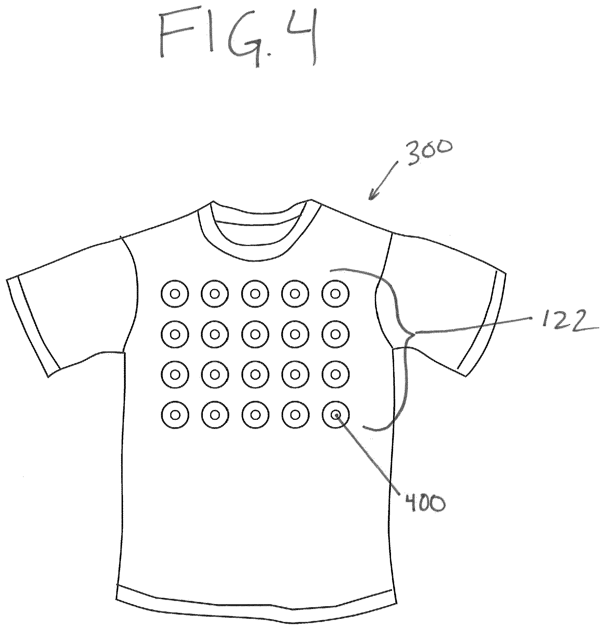

Fig. 4 shows another example of the garment 300. The garment shown in Fig. 4 is similar to the garment shown in Fig. 3 except each of the electrodes 122 additionally comprises a magnetic resonance fiducial marker 400. The magnetic resonance fiducial marker 400 may for example be a coil tuned to a particular frequency or contain a substance which readily shows up on magnetic resonance imaging scan. The magnetic resonance fiducial marker 400 may be used to effectively localize the position of each of the electrodes 122 in the three-dimensional image data. This may help increase the accuracy of the resistive model.

A precise knowledge of the electrophysiology of the heart may be important for therapy planning. The best way to get this information is by a catheter procedure. Here, a catheter is inserted into the heart and a contact at the tip of the catheter maps the electrical potential. An alternative is to map the electric and/or magnetic field at the surface of the patient. Together with the electrical conductivity of the patient and a reasonable model of where the electrical sources can lie, a reconstruction of the potentials can be computed. A guess for the patient's conductivity can be reached by medical imaging (CT, MRI) and an appropriate segmentation.

The main draw-back of the gold standard, the use of a catheter, is the invasiveness. The other methods lack accuracy. One component of the inaccuracy is the lack of precise knowledge of the conductivity. There are two components that contribute to this accuracy. First, the segmented patient still does not resemble the actual patient fully and second, there is patient movement between the segmentation and the actual recording of the ECG data.

In some examples, the MRI system may be a Special MRI system, such as a ZTE system, that is capable to ramp the magnetic field in field up and down. The Larmor frequency may be low enough that for this frequency the capacitive effect of the cell membrane for the current transport becomes small but high enough that nerve stimulation is not much of an issue if a current is applied to the patient. A frequency range between 20 kHz and 200 kHz is one range that will work effectively. On the surface of the patient, electrodes are placed. Through these electrodes, currents are feed (patient can tolerate about 1 A/m² at these frequencies) for approximately 100 ms. This generates a flip angle of about 10° (depending on the local current density). The field is ramped up as fast as possible and MRI data are encoded and recorded (resolution about 1 cm). From many such images, using different positions of the current sources, the current distribution and ultimately the conductivity is derived. During idle times in the scan, ECG data are recorded (in the very low noise environment of a MRI machine). The ZTE scanner also provides an anatomical picture for still needed conductivity corrections and the placement of the virtual current sources in the heart. With all the data, the electrical potential distribution in the heart can be predicted.

The basis of some examples is the ability in an MRI system to ramp the field down to low levels. At this very low field, the interaction of electric currents and proton spins can be used to determine the patient's conductivity. For this, a quite low strength (e.g. 0.5 T) superconducting magnet is needed. Such a magnet could be operated while being permanently connected to an external power supply. There will be some thermal load to the high temperature stage of the cryo-machine. With a proper design of the leads, the power load could be below 10 W. The stored energy in such a magnet could be around 200 kJ. With a peak current of say 200 A, this means the inductivity is in the order of 10 H. The maximum voltage at the terminals may be in the order of 400 V, so the ramping time from zero to 0.5 T should be in the order of 5 seconds. This is much too slow for any reasonable fast field switching operation in the scanner. Therefore there are two MRI operation modes. One operates at 0.5 Tesla and the other at e.g. 50 mTesla. So there is a 50 mTesla RF and receive system e.g. as an insert system only for the electrophysiology application. For the field homogeneity, the shim irons shall be operated in their linear regime, i.e. not in saturation. At 0.5 Tesla this can be achieved by proper shaping of the shim iron. The gradient system does not need any alteration. In this system an amplifier (current source) in needed to operate the man (superconducting magnet). A single 400 V, 200 A system would do the job. However, it may be more economic to use a 400V, 40 A amplifier and a 10V, 200A amplifier.

An additional component that may be used with some examples for the electrophysiology system is a "vest" or garment with many electrodes attached to the patient. The electrodes may cover virtually all the available chest area. This may have the advantage of avoiding current hot spots, as a high current shall pass through the patient. The electrodes may be connected to a current generator that is able to apply voltages to the electrodes at frequencies e.g. between 20 and 200 kHz. The electrodes have a connection to a low frequency amplifier to be able to measure the ECG signals. It would be possible to construct a system that can measure ECG signal even while sending the kHz signals. However, this is costly and it is sufficient that sending and receiving are alternated.

In some examples the procedure to acquire magnetic resonance data may incorporate one or more of the following steps:
If necessary the scanner is set to the low field strength mode (Low current high voltage amplifier connects).

The field is set to the highest possible value. This value is determined by the wanted time when the almost zero field value has to be reached. This means the closer to the target time, the lower the field strength, i.e. a ramp is executed.

After reaching the target field strength (e.g. 2 mTesla) a current is applied through the electrodes. Almost all electrodes are used as current sources or sinks (currents only between two electrodes are less efficient). The current is held for a defined time period (e.g. 200 ms).

The field is ramped to the field strength used for the MRI experiment (e.g. 50 mTesla) with the highest possible slew rate.

A gradient sequence (e.g. EPI, radial) is applied and the MRI signals are recorded. Preferably, each sequence results in a fully encoded image. This is possible due to the quite large voxel size (about 1 cm).

Optionally, a spin echo pulse is applied to refocus the signal (which means that a low frequency sending RF send coil is needed. Otherwise only a receive coil system is necessary).
Then the procedure is repeated. This may mean that the field is increased before the next low field current pulse is applied. After a full volume is encoded, a different current pattern is applied.

The low field time point may be determined using ECG triggering. The cardiac cycle is analyzed and the next desired heart phase time point is predicted. As the ventilation status of the lung also alters current paths, it is recorded, too.

During all times, where technically possible, The ECG of the patient is recorded. The ECG signal is altered by movement status of the patient but also due to the applied magnetic field (magneto-hydrodynamic effect). At this low field strength, the effect is low. Nevertheless a correction must be applied. If not sufficient ECG data can be recorded during the MRI procedure, additional time in the scanner is reserved for it.

In the scanner and ideally also during the scan time, electrical impedance tomography data are recorded. This may be done at the MRI Larmor-frequency and lower (and possibly higher) frequencies. Some data may be recorded during the application of the RF pulse by switching some electrodes to receive mode.

In some examples, in the scanner in the same position, a high quality MRI image (high field) is acquired, too.

In the reconstruction step, the best fit for the (anisotropic/frequency dependent) conductivity is determined. The simplest way would be to assign conductivity to the voxels and vary the conductivity until the measured data are best fit. After finishing the conductivity model using MRI and EIT data, current sources (time dependent and moving due to heart motion) are placed in the model and varied to gain the best fit of the ECG data. How this is done precisely is known in the (patent) literature and not part of this invention.

Examples may be realized using a ZTE MRI scanner. Nevertheless, it is possible to build a MRI scanner only for this task. This scanner would not need to use superconducting coils, but would not be able to acquire high quality MRI images.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 102: magnet
- 104: magnet power supply
- 106: bore of magnet
- 108: imaging zone
- 109: region of interest
- 110: magnetic field gradient coils
- 112: magnetic field gradient coil power supply
- 114: radio-frequency coil
- 116: transceiver
- 118: subject
- 120: subject support
- 122: electrodes - first portion
- 122': electrodes - second portion
- 124: current source
- 126: computer system
- 128: hardware interface
- 130: processor
- 132: user interface
- 134: computer memory
- 140: machine executable instructions
- 142: pulse sequence commands
- 142': readout gradient commands
- 144: known or measured RF current
- 146: magnetic resonance data
- 148: three-dimensional image data
- 150: resistive model
- 152: first finite differenc model
- 154: current flow mapping
- 156: second finite difference model
- 200: set the average magnetic field strength within the imaging zone to a first value by controlling the magnet power supply with the pulse sequence commands
- 202: set the average magnetic field strength within the imaging zone to a second value by controlling the magnet power supply with the pulse sequence commands
- 204: control the current source to have a known RF current travel between the first portion of the electrodes and the second portion of the electrodes
- 206: acquire the magnetic resonance data from the subject by controlling the magnetic resonance imaging system with the readout gradient commands
- 208: reconstruct three-dimensional image data from the magnetic resonance data
- 210: calculate a resistive model of the subject using the three-dimensional image data and the known RF current through the electrodes
- 300: garmant
- 400: Magnetic resonace fiducial marker

## Claims

1. A magnetic resonance imaging (100) system comprising:
a main magnet (102) with an imaging zone (108), wherein the main magnet is configured for generating a main magnetic field with an average magnetic field strength within the imaging zone;
a gradient magnetic field system (110, 112) for generating a spatially dependent gradient magnetic field within the imaging zone;
a magnet power supply (104) configured for adjusting the average magnetic field strength within the imaging zone;
a current source (124) for supplying RF current between multiple electrodes (122, 122'), wherein the multiple electrodes comprise a first portion (122) and a second portion (122'), wherein the current source is configured for supplying the RF current between the first portion and the second portion, wherein the multiple electrodes are configured for forming an electrical contact with an exterior surface of a subject (118);
a memory (134) containing machine executable instructions (140) and pulse sequence commands (142), wherein the pulse sequence commands comprise instructions for controlling the magnetic resonance imaging system for acquiring magnetic resonance data (146) from the imaging zone according to a three-dimensional imaging protocol, wherein the pulse sequence commands comprise readout gradient commands (142') for controlling the gradient magnetic field system;
a processor (130) for controlling the magnetic resonance imaging system;
wherein execution of the machine executable instructions cause the processor to:
set (200) the average magnetic field strength within the imaging zone to a first value by controlling the magnet power supply with the pulse sequence commands;
set (202) the average magnetic field strength within the imaging zone to a second value by controlling the magnet power supply with the pulse sequence commands, the second value is lower than the first value;
control (204) the current source to have a known RF current (144) travel between the first portion of the electrodes and the second portion of the electrodes;
acquire (206) the magnetic resonance data from the subject by controlling the magnetic resonance imaging system with the readout gradient commands;
reconstruct (208) three-dimensional image data (148) from the magnetic resonance data; and
calculate (210) a resistive model (150) of the subject using the three-dimensional image data and the known RF current through the electrodes.

2. The magnetic resonance imaging system of claim 1, wherein the magnetic resonance imaging system is configured for receiving an ECG signal from the subject, wherein execution of the machine executable instructions further comprises calculating a heart electrical potential of the subject using the resistive model, the ECG signal, and an electrical source model.

3. The magnetic resonance imaging system of claim 2, wherein the execution of the readout gradient commands is at least partially triggered by the ECG signal.

4. The magnetic resonance imaging system of any one of the preceding claims, wherein the current source comprises a current sensor to individually measure an RF electrode current for each of the multiple electrodes, wherein the known RF current is determined using the RF electrode current for each of the multiple electrodes.

5. The magnetic resonance imaging system of claim 4, wherein the resistive model is calculated using a first finite difference model (152) and a second finite difference model (156), wherein the first finite difference model is configured for solving for a current flow (154) through the subject using the RF electrode current for each of the multiple electrodes and the amplitude of the three-dimensional image data, wherein the first finite difference model is configured for calculating the current flow through the subject using a first optimization algorithm to optimize a first objective function, wherein the first objective function fits the current flow to the intensity of the three-dimensional image data using the Biot-Savat law, wherein the second finite difference model fits the resistive model to the current flow using a second objective function, and wherein the second objective function fits the resistive model to the current flow using Ohm's law and the RF electrode current for each of the multiple electrodes.

6. The magnetic resonance imaging system of claim 5, wherein the current source is configured for switching the multiple electrodes between the first portion and the second portion, wherein the machine executable instruction further cause the processor to reconstruct the three-dimensional image data for multiple permutations of the multiple electrodes distributed between the first portion and the second portion, wherein the first objective function and the second objective function combine data from the multiple permutations of the multiple electrodes.

7. The magnetic resonance imaging system of claim 5 or 6, wherein execution of the machine executable instructions further cause the processor to control the current source to acquire electrical impedance tomography data using the multiple electrodes, wherein the second objective function further fits the resistive model to the electrical impedance tomography data.

8. The magnetic resonance imaging system of any one of the preceding claims, wherein the magnetic resonance imaging system further comprises a garment (300), and wherein the garment comprises the multiple electrodes.

9. The magnetic resonance imaging system of any one of the preceding claims, wherein the pulse sequence commands are any one of the following: a spin echo pulse sequence commands, gradient echo pulse sequence commands, ZTE pulse sequence commands, EPI pulse sequence commands, radially samples pulse sequence commands, and pulse sequence commands with comprising a spiral read out gradient sequence.

10. The magnetic resonance imaging system of any one of the preceding claims, wherein the second value of the average magnetic field strength is chosen such that the Larmor frequency is between 20 kHz and 200kHz.

11. The magnetic resonance imaging system of any one of the preceding claims, wherein any one of the following:
the the second value of the average magnetic field strength is between 5 mTesla and 0.2 mTesla;.
execution of the machine executable instructions cause the processor to maintain the average magnetic field strength within the imaging zone at the first value for any one of the following: at least 10 ms, at least 20 ms, at least 100 ms, at least 300 ms, and at least 500 ms before setting the average magnetic field strength within the imaging zone to the second value; and
combinations thereof.

12. The magnetic resonance imaging system of any one of the preceding claims, wherein execution of the machine executable instructions cause the processor to perform any one of the following:
setting (204) the average magnetic field strength within the imaging zone to a third value by controlling the magnet power supply with the pulse sequence commands before acquiring the magnetic resonance data from the subject by controlling the magnetic resonance imaging system with the readout gradient commands, wherein the thrid value is lower than the first value, wherein the third value is higher than the second value; and
acquire the magnetic resonance data from the subject by controlling the magnetic resonance imaging system with the readout gradient commands while the average magnetic field strength within the imaging zone is set at the second value.

13. The magnetic resonance imaging system of any one of the preceding claims, wherein any one of the following:
the magnetic resonance imaging system is further configured for receiving a respiratory signal, wherein the acquisition of the magnetic resonance data is at least partially triggered by the respiratory signal;
the multiple electrodes comprise magnetic resonance fiducial markers (400), wherein execution of the machine executable instructions further causes the processor to register a location of each of the multiple electrodes to the three-dimensional image data by detecting a fiducial marker signal in the the three-dimensional image data, wherein the resistive model is further calculated using the location of each of the multiple electrodes; and
combinations thereof.

14. A computer program product comprising machine executable instructions (140) for execution by a processor (130) controlling a magnetic resonance imaging system (100), wherein the magnetic resonance imaging system comprises a main magnet (102) with an imaging zone (108), wherein the main magnet is configured for generating a main magnetic field with an average magnetic field strength within the imaging zone, wherein the magnetic resonance imaging system further comprises a gradient magnetic field system (110, 112) for generating a spatially dependent gradient magnetic field within the imaging zone, wherein the magnetic resonance imaging system further comprises a magnet power supply (104) configured for adjusting the average magnetic field strength within the imaging zone, wherein the magnetic resonance imaging system further comprises a current source (124) for supplying RF current between multiple electrodes (122, 122'), wherein the multiple electrodes comprise a first portion (122) and a second portion (122'), wherein the current source is configured for supplying the RF current between the first portion and the second portion, wherein the multiple electrodes are configured for forming an electrical contact with an exterior surface of a subject (118),
wherein execution of the machine executable instructions cause the processor to:
set (200) the average magnetic field strength within the imaging zone to a first value by controlling the magnet power supply with pulse sequence commands (142), wherein the pulse sequence commands comprise instructions for controlling the magnetic resonance imaging system for acquiring magnetic resonance data from the imaging zone according to a three-dimensional imaging protocol, wherein the pulse sequence commands comprise readout gradient commands (142') for controlling the gradient magnetic field system;
set (202) the average magnetic field strength within the imaging zone to a second value by controlling the magnet power supply with the pulse sequence commands, the second value is lower than the first value;
control (204) the current source to have a known RF current (144) travel between the first portion of the electrodes and the second portion of the electrodes;
acquire (206) the magnetic resonance data from the subject by controlling the magnetic resonance imaging system with the readout gradient commands;
reconstruct (208) three-dimensional image data (148) from the magnetic resonance data; and
calculate (210) a resistive model (150) of the subject using the three-dimensional image data and the known RF current through the electrodes.

15. A method of operating a magnetic resonance imaging system (100), wherein the magnetic resonance imaging system comprises a main magnet (102) with an imaging zone (108), wherein the main magnet is configured for generating a main magnetic field with an average magnetic field strength within the imaging zone, wherein the magnetic resonance imaging system further comprises a gradient magnetic field system (110, 112) for generating a spatially dependent gradient magnetic field within the imaging zone, wherein the magnetic resonance imaging system further comprises a magnet power supply (104) configured for adjusting the average magnetic field strength within the imaging zone, wherein the magnetic resonance imaging system further comprises a current source (124) for supplying RF current between multiple electrodes (122, 122'), wherein the multiple electrodes comprise a first portion (122) and a second portion (122'), wherein the current source is configured for supplying the RF current between the first portion and the second portion, wherein the multiple electrodes are configured for forming an electrical contact with an exterior surface of a subject, wherein the method comprises:
setting (200) the average magnetic field strength within the imaging zone to a first value by controlling the magnet power supply with pulse sequence commands (142), wherein the pulse sequence commands comprise instructions for controlling the magnetic resonance imaging system for acquiring magnetic resonance data from the imaging zone according to a three-dimensional imaging protocol, wherein the pulse sequence commands comprise readout gradient commands (142') for controlling the gradient magnetic field system;
setting (202) the average magnetic field strength within the imaging zone to a second value by controlling the magnet power supply with the pulse sequence commands, the second value is lower than the first value;
controlling (204) the current source to have a known RF current (144) travel between the first portion of the electrodes and the second portion of the electrodes;
acquiring (206) the magnetic resonance data from the subject by controlling the magnetic resonance imaging system with the readout gradient commands;
reconstructing (208) three-dimensional image data (148) from the magnetic resonance data; and
calculating (210) a resistive model (150) of the subject using the three-dimensional image data and the known RF current through the electrodes.
